# EUROPEAN PATENT SPECIFICATION

(11) **EP 1 809 739 B1**
(45) Date of publication and mention of the grant of the patent: **15.10.2014**
(21) Application number: 05775294.1
(22) Date of filing: 13.07.2005
(51) Int. Cl.: C12N 5/0735

(54) **MEDIUM FOR GROWING HUMAN EMBRYONIC STEM CELLS**
WACHSTUMSMEDIUM FÜR MENSCHLICHE EMBRYONALE STAMMZELLEN
MILIEU DESTINE A LA CROISSANCE DE CELLULES SOUCHES EMBRYONNAIRES HUMAINES

(30) Priority: 13.07.2004 US 587843 P; 01.03.2005 US 657990 P
(43) Date of publication of application: 25.07.2007
(60) Divisional application: 10180759.2
(73) Proprietor: Asterias Biotherapeutics, Inc., Menlo Park, CA 94025 (US)
(72) Inventor: LI, Yian, Menlo Park, CA 94025 (US); MANDALAM, Ramkumar, Union City, CA 94587 (US)
(74) Representative: Bassil, Nicholas Charles
(86) International application number: PCT/US2005/025179
(87) International publication number: WO 2006/017370

(56) References cited:
- WO-A-03/020920
- WO-A-2004/055155
- AMIT M ET AL: "Feeder layer- and serum-free culture of human embryonic stem cells" BIOLOGY OF REPRODUCTION, vol. 70, March 2004 (2004-03), pages 837-845, XP002978624 ISSN: 0006-3363
- WANG GUANGWEN ET AL: "Noggin and bFGF cooperate to maintain the pluripotency of human embryonic stem cells in the absence of feeder layers" BIOCHEMICAL AND BIOPHYSICAL RESEARCH COMMUNICATIONS, vol. 330, no. 3, 13 May 2005 (2005-05-13), pages 934-942, XP004833728 ISSN: 0006-291X
- XU REN-HE ET AL: "Basic FGF and suppression of BMP signaling sustain undifferentiated proliferation of human ES cells" NATURE METHODS, vol. 2, no. 3, March 2005 (2005-03), pages 185-190, XP002340999 ISSN: 1548-7091
- KLIMANSKAYA I ET AL: "Human embryonic stem cells derived without feeder cells" THE LANCET, vol. 365, no. 9471, 7 May 2005 (2005-05-07), pages 1636-1641, XP004882308 ISSN: 0140-6736
- LI YAN ET AL: "Expansion of human embryonic stem cells in defined serum-free medium devoid of animal-derived products" BIOTECHNOLOGY AND BIOENGINEERING, vol. 91, no. 6, 20 September 2005 (2005-09-20), pages 688-698, XP002351170 ISSN: 0006-3592
- THOMSON J.A. ET AL: "Embryonic Stem Cell Lines Derived from Human Blastocysts", SCIENCE, vol. 282, 6 November 1998 (1998-11-06), pages 1145-1147,
- THE BUSINESS JOURNAL, vol. 17, no. 31, 28 April 2000 (2000-04-28), page 43,
- GULBRANDSEN, CARL: 'Declaration' 02 March 2009,
- "1998 Information for Contributors", SCIENCE, vol. 279, 2 January 1998 (1998-01-02), page 108,
- VOGEL G.: 'Wisconsin to Distribute Embryonic Cell Lines' SCIENCE, [Online] vol. 287, no. 5455, pages 948 - 949 Retrieved from the Internet: <URL:http://www.sciencemag.org/cgi/content/ full/287/5455/948>
- VOGEL G.: "Researchers Get Green Light for Work on Stem Cells", SCIENCE, [Online] vol. 289, no. 5484, 1 September 2000 (2000-09-01), pages 1442-1443, Retrieved from the Internet: URL:http://www.sciencemag.org/cgi/content/ full/289/5484/1442>
- WERTZ D C: "Embryo and stem cell research in the United States: history and politics", GENE THERAPY, vol. 9, no. 11, June 2002 (2002-06), pages 674-678,
- "[excerpts]" In: Kirschtein R & Skirboll L R: "Stem Cells: Scientific progress and furture research directions", June 2001 (2001-06), National Institutes of Health
- REUBINOFF B E ET AL.: "Embryonic stem cell lines from human blastocysts: somatic differentiation in vitro", NATURE BIOTECHNOLOGY, vol. 18, no. 4, April 2000 (2000-04), pages 399-404,
- OWEN-SMITH J & MCCORMICK J: "An international gap in human ES cell research", NATURE BIOTECHNOLOGY, vol. 24, no. 4, April 2006 (2006-04), pages 391-392,
- ROBERTSON D: "NIH sacrifices commercial rights in WiCell deal", NATURE BIOTECHNOLOGY, vol. 19, no. 11, 1 November 2001 (2001-11-01), page 1001, XP007915638, ISSN: 1087-0156

## Description

### BACKGROUND

Regenerative medicine is benefiting from recent advances relating to the isolation, culture, and use of various types of progenitor cells. This disclosure provides further improvements for the commercial development of human pluripotent stem cells and their derivatives.

Embryonic stem cells have two very special properties: First, unlike other normal mammalian cell types, they can be propagated in culture almost indefinitely, providing a virtually unlimited supply. Second, they can be used to generate a variety of tissue types of interest as a source of replacement cells and tissues for use in tissue therapy, or for use in the screening of pharmaceutical agents.

Thomson et al. (U.S. Patent 5,843,780; Proc. Natl. Acad. Sci. USA 92:7844, 1995) were the first to successfully Isolate and propagate pluripotent stem cells from primates. They subsequently derived human embryonic stem (hES) cell lines from human blastocysts (Science 282:114, 1998). Gearhart and coworkers derived human embryonic germ (hEG) cell lines from fetal gonadal tissue (Shamblott et al., Proc. Natl. Acad. Sci. USA 95:13726, 1998; and U.S. Patent 6,090,622). Both hES and hEG cells have the long-sought characteristics of pluripotent stem cells: they can be cultured extensively without differentiating, they have a normal karyotype, and they are capable of producing a number of important cell types.

A significant challenge to the use of pluripotent stem cells for therapy is that they are traditionally cultured on a layer of feeder cells to prevent differentiation (U.S. 5,843,780; U.S. 6,090,622). According to Thomson et al. (Science 282:114, 1998), hPS cells cultured without feeders soon die, or differentiate into a heterogeneous population of committed cells. Leukemia inhibitory factor (LIF) inhibits differentiation of mouse ES cells, but it does not replace the role of feeder cells in preventing differentiation of human ES cells.

U.S. Patent 6,800,480 (Geron Corp.) is entitled *Methods and materials for the growth of primate-derived primordial stem cells.* International Patent Publication WO 01/51616 (Geron Corp.) is entitled *Techniques for growth and differentiation of human pluripotent stem cells.* An article by Xu et al. (Nature Biotechnology 19:971, 2001) is entitled *Feeder-free growth of undifferentiated human embryonic stem cells.* An article by Lebkowski et al. (Cancer J. 7 Suppl. 2:S83, 2001) is entitled *Human embryonic stem cells: culture, differentiation, and genetic modification for regenerative medicine applications.* These publications report exemplary culture reagents and techniques for propagating embryonic stem cells in an undifferentiated state, and their use In preparing cells for human therapy.

WO 03/020920 describes expansion of hES cells in an undifferentiated state without using feeder cells using a medium comprising serum, bFGF, optionally in combination with other growth factors such as SCF or Flt3L and basement membrane components such as MatrigelTM or laminin. Amit et al. (Biology of Reproduction 70:837,2004) and WO 2004/055155 describe feeder-free and serum-free culture of hES using a system based on serum replacement, TGFbeta1, LIF, bFGF, and a fibronectin matrix. Wang et al. (Blochem, Biophys. Res. Acta. 330(3): 934, 2005) and Xu et al. (Nature Methods 2(3): 185 (2005) describe the use of bFGF and noggin to maintain hES cells in an undifferentiated state. Klimanskaya et al. (The Lancet 365(9471): 1636 2005) describes the establishment of a hES cell line In feeder-free conditions using bFGF, LIF and extracellular matrix components. Yan et al. (Biotechnology & Bioengineering 91(6):688 2005) describes the culture of hES cells in an undifferentiated state in a medium comprising bFGF. Vogel, G (Science 287(5455):948, 2000; and 289(5484): 1442, 2000) report the distribution of hES cell lines by WARF.

The new technology described in this disclosure will facilitate growing and manipulating undifferentiated pluripotent stem cells, and help realize the full commercial potential of embryonic cell therapy.

### SUMMARY OF THE INVENTION

This disclosure provides a medium for culturing human embryonic stem (hES) cells *in vitro* such that they proliferate without differentiating as defined the claims. The technology allows the user to rapidly produce high-quality embryonic stem cells for use in therapy and drug discovery, in a cost-effective and controlled manner, from defined or commercially available reagents.

Application of the technology involves introducing human embryonic stem cells into a culture environment containing components described and exemplified in more detail in the sections that follow. Typically, the environment will contain a support structure such as an extracellular matrix, a culture medium as defined in the claims, and one or more factors added to the medium that support proliferation of the pPS cells in an undifferentiated state.

The culture media comprise an isotonic buffer, a protein or amino acid source, lipids and hormones, and may also comprise nucleosides or nucleotides as defined in the claims. A factor included in the medium is fibroblast growth factor at a concentration of at least 40 ng/ml. It has been discovered that high concentration FGF in a suitable medium is sufficient to maintain pPS cells in a substantially undifferentiated state through extended culture. Other factors listed in this disclosure can be added to improve the quality and expansion rate of the culture when desired. The media may be made from defined components, wherein all protein in the medium is human. This means that each protein is either isolated from a human tissue or fluid, or has been recombinantly produced by expressing a suitably adapted human gene sequence.

This invention Is a medium suitable for commercial distribution that can be used for culturing pPS cells in vitro such that they proliferate without differentiating. Such a tissue culture medium may be either ready for culture, or in concentrated, powdered, or component form that are diluted or combined prior to culture. Medium components include an isotonic buffer, protein nutrient, lipids, and growth factors, as defined the claims including a high concentration of fibroblast growth factor (FGF) of at least 40ng/ml. Other possible ingredients are listed in the Detailed Description and Examples. As described below, the medium and other ingredients of the culture environment can be defined and derived from human and/or synthetic sources, avoiding the use of non-human animal products entirely.

### DRAWINGS

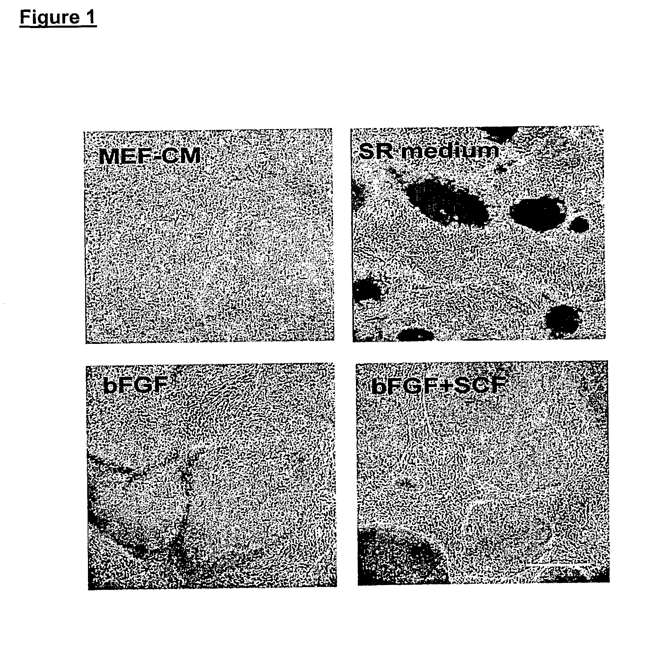

**Figure 1** shows hES cells maintained for 6 passages in medium conditioned by mouse embryonic fibroblasts (mEF-CM), fresh SR medium alone, or fresh medium supplemented with 40 ng/mL bFGF or bFGF with stem cell factor. bar = 800 µm. The presence of bFGF at 40 ng/mL in fresh medium was sufficient to maintain the undifferentiated phenotype in a manner comparable to conditioned medium. Cells grown in fresh SR medium unsupplemented with FGF showed substantial differentiation.

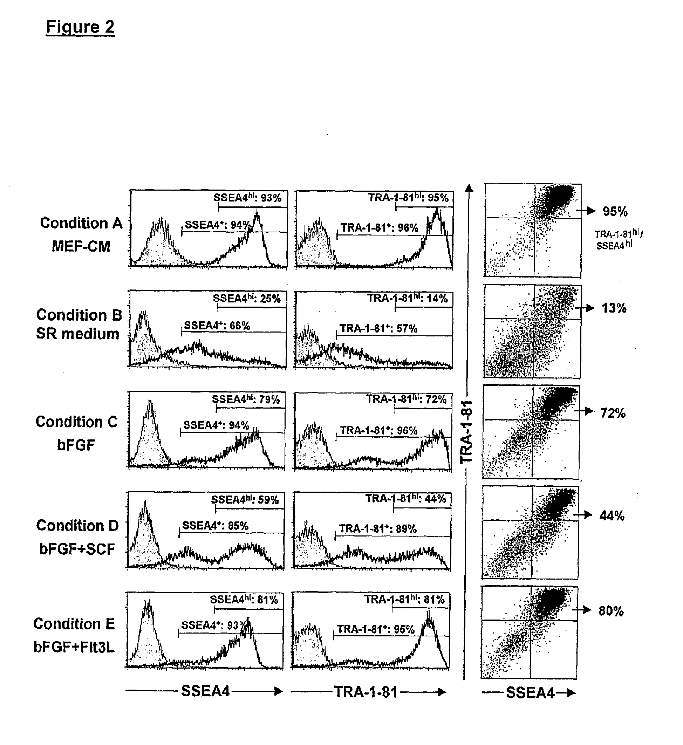

**Figure 2** shows that a high proportion of cells grown in fresh medium maintain the undifferentiated phenotype, as measured by high-level expression of SSEA-4 and Tra-1-81.

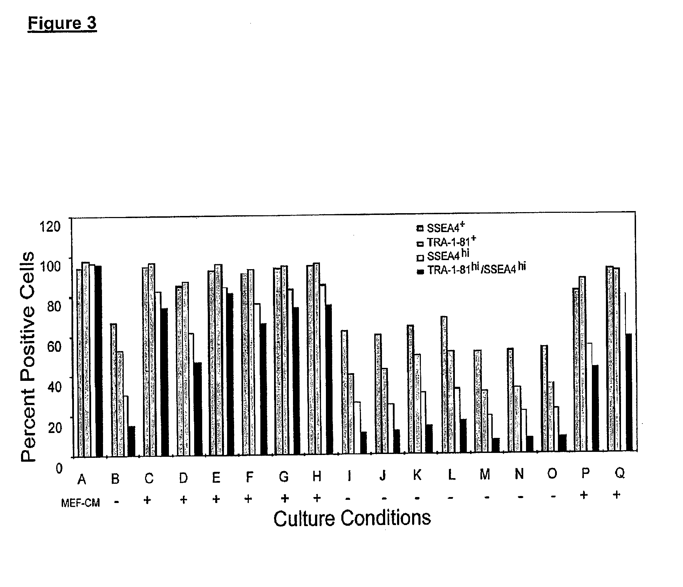

**Figure 3** compares SSEA-4 and Tra-1-81 expression amongst different growth conditions Media containing high FGF (marked by "+") maintained the undifferentiated phenotype comparable to cells grown in mEF conditioned medium; whereas combinations of growth factors without FGF (marked by "-") showed evidence of differentiation.

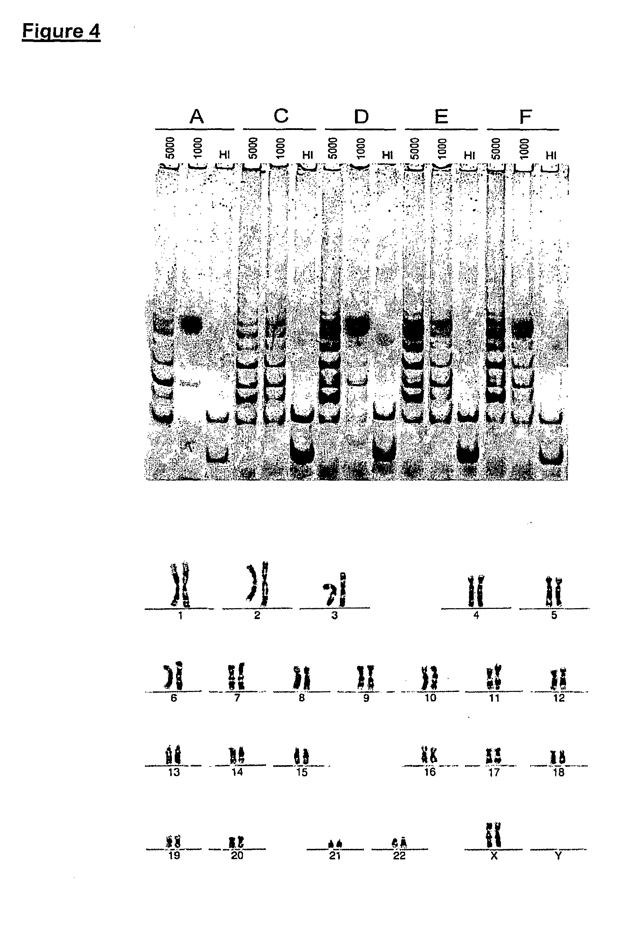

**Figure 4** shows that cells grown in high bFGF concentration are positive for telomerase activity (Panel A), and have a normal karyotype (the H7 cell line is female).

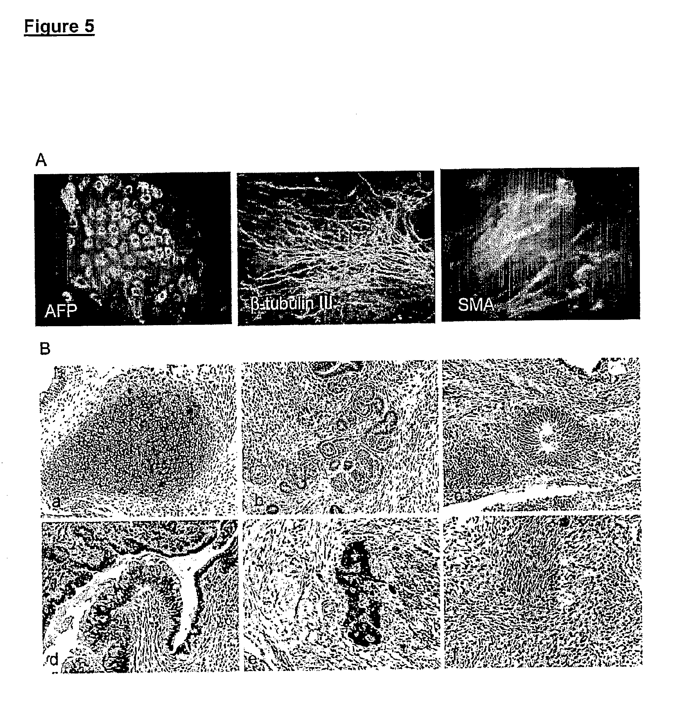

**Figure 5** shows that H7 hES cells maintained in high levels of bFGF that formed differentiated cells in tissue culture (Panel A), or generated teratomas when injected to SCID/beige mice (Panel B). Cells grown in fresh medium clearly retained their ability to differentiate into cells representing all three germ layers.

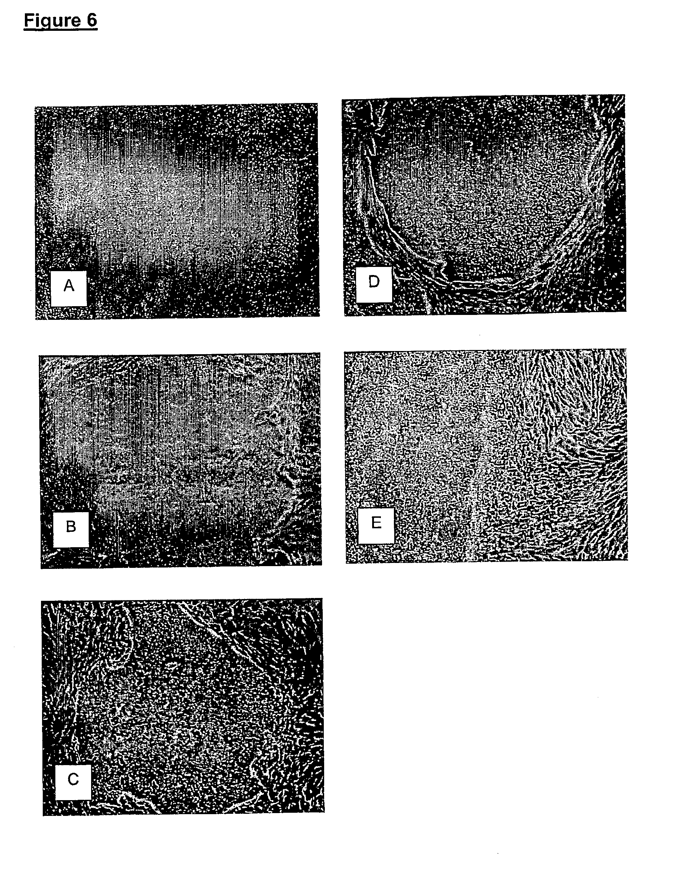

**Figure 6** shows colonies of hES cells after six passages (sufficient for full adaptation) in different base media. (A) mEF conditioned ES medium + bFGF (8 ng/mL); (B) X-VIVO 10™ Serum-free Medium (hereinafter "X-VIVO 10") + bFGF (40 ng/mL); (C) X-VIVO 10 + bFGF (40 ng/mL) + stem cell factor (SCF, Steel factor) (15 ng/mL); (D) X-VIVO 10 + bFGF (40 ng/mL) + Flt3 ligand (75 ng/mL); (E) OBSF-60™ Serum Free Medium (hereinafter QBSF-60) + bFGF (40 ng/mL). All three base media (ES medium, X-VIVO 10, and OBSF™-60) can be used to expand hES cells in feeder-free culture. In this illustration, the cells growing in combination shown in (C) expanded 8.2-fold per passage, whereas those in conditioned medium expanded 2.2-fold. The use of suitable fresh medium causes rapid expansion of undifferentiated hES cells.

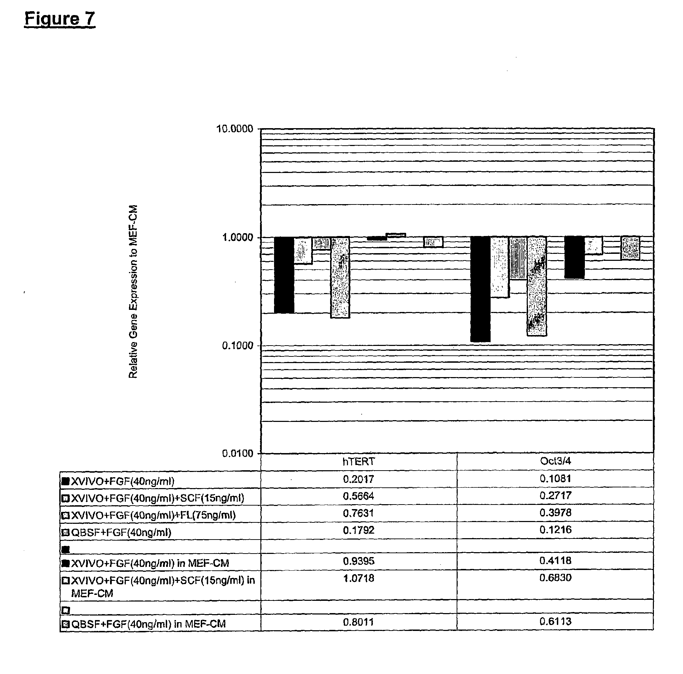

**Figure 7** shows the gene expression profile of hTERT and Oct3/4, measured by real time RT-PCR, as described in Example 5.

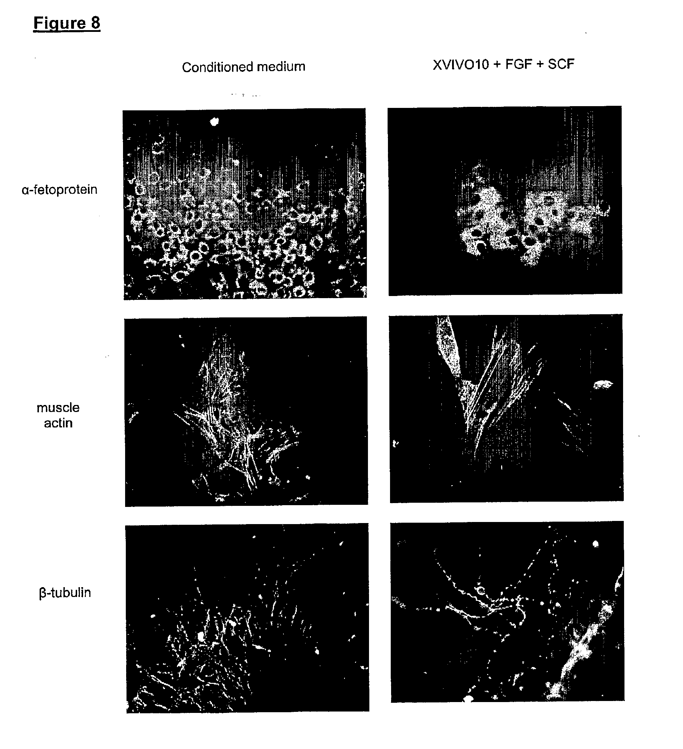

**Figure 8** demonstrates that cells cultured in unconditioned medium retain their pluripotency. hES cells passaged 7 times in mEF conditioned medium, or unconditioned X-VIVO 10 medium containing bFGF and SCF. The cells were then differentiated into embryoid bodies, plated, and analyzed by immunocytochemistry for phenotypic markers representing each of the three germ layers. The cells stain for α-fetoprotein (representing endoderm); muscle actin (representing mesoderm), and β-tubulin III (representing ectoderm). The cells grown in the culture system described in this patent application are suitable for making a wide scope of differentiated cell types.

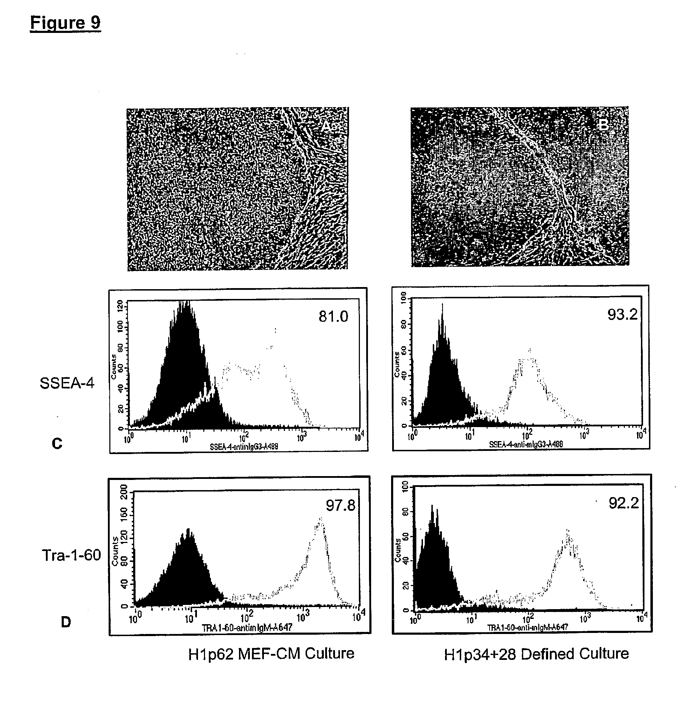

**Figure 9** shows the morphology of undifferentiated hES cell cultures growing in mEF conditioned medium on BD Matrigel™ Basement Membrane Matrix (hereinafter "Matrigel") (Panel A), compared with cultures growing in defined medium containing 80 ng/mL FGF on laminin (Panel B). Also shown is a comparison of the hES cell markers SSEA-4 (Panel C) and Tra-1-60 (Panel D). Culture performance in the defined medium on laminin was superior: very large ES cell colonies were observed, representing ~80% of the culture.

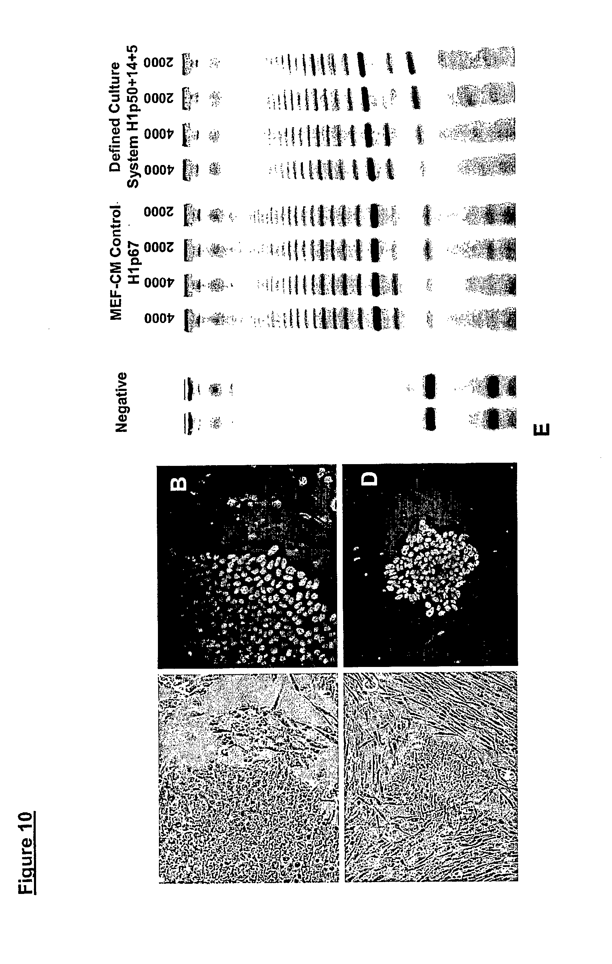

**Figure 10** shows cultures of hES cells growing in defined medium on laminin by phase contrast (Panels A and C) and by staining for Oct 3/4 expression (Panels B and D). Panel E shows TRAP analysis for telomerase activity. BY all these criteria, hES cells grown in medium containing 80 ng/mL have all the characteristics of undifferentiated pluripotent cells.

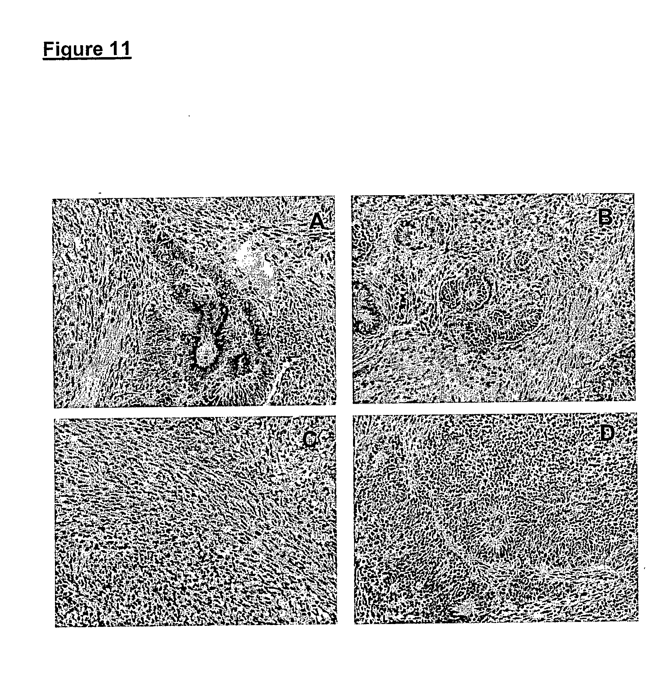

**Figure 11** shows teratomas derived from hES cells grown in these conditions (200X). Panel A: Pigmented epithelium (endoderm); Panel B: Renal tissue (endoderm); Panel C: Mesenchymal tissue (mesoderm); Panel D: Neural tubes (ectoderm) - representing all three germ layers.

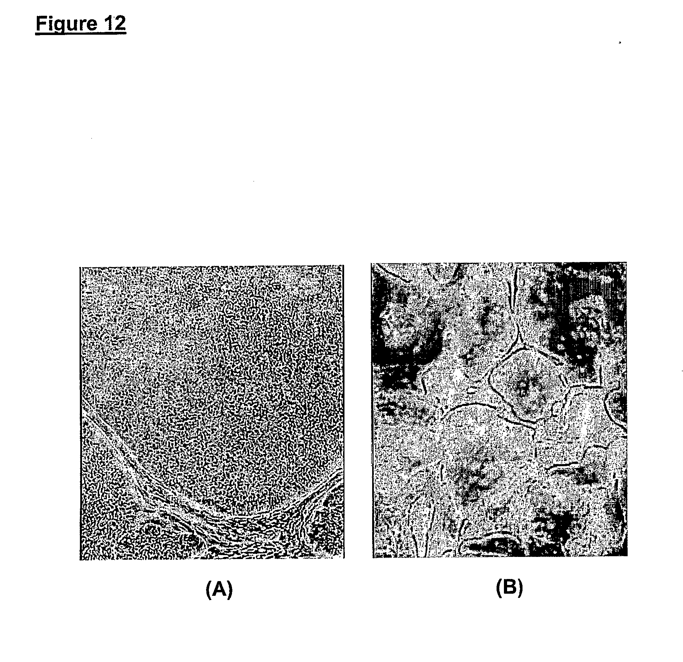

**Figure 12** shows the culture morphology of undifferentiated hES cells grown in X-VIVO 10 + 80 ng/ml hbFGF + 0.5 ng/ml TGFβ1 on Matrigel-GFR. (A) H1p46+9 cells, 4X magnification; (B) H1p46+9 cells, 1X magnification.

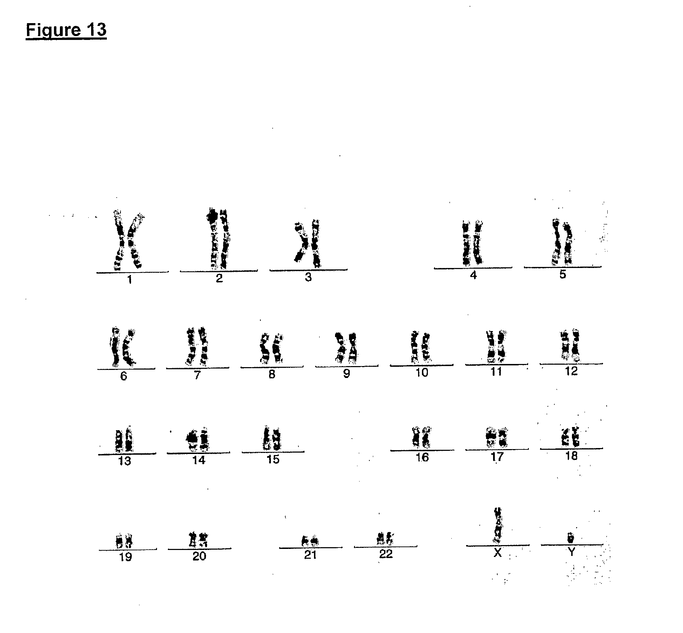

**Figure 13** shows the karyotype of undifferentiated H1p46+8 hES cells grown in X-VIVO 10 + 80 ng/ml hbFGF + 0.5 ng/ml TGFβ1 on Matrigel-GFR.

### DETAILED DESCRIPTION

Previous technology for growing primate pluripotent stem (pPS) cells has required that the cell culture environment contain cells or cell supernatants from other species to inhibit differentiation while they proliferate. This requirement complicates commercialization and therapeutic use of these cells, because the reagents are not easy to standardize, and constitute a potential source of contamination.

This disclosure provides an improved medium for culturing hES cells *in vitro* as defined In the claims without requiring a layer of feeder cells to support the culture - either in direct contact with the hES cells, or for preconditioning the medium in which the hES cells are cultured.

As a result of thorough investigation of the features required, it has now been determined that the beneficial effect of the feeder cells can be replaced by providing a mixture of soluble factors. It turns out that the presence of feeder cell components is not required, as long as the signal transduction pathways required for undifferentiated growth are adequately activated by factors in the culture environment.

This disclosure shows *inter alia* how hES cell lines can be derived and grown in medium that has not been preconditioned, but has been supplemented with ingredients that perform essentially the same function as factors secreted from feeder cells. Certain factor combinations comprising moderate to high levels of fibroblast growth factors at a concentration of at least 40 ng/ml and other cells generate cultures that can proliferate 20-fold or more through 6 or more passages, while maintaining a majority of the cells in the culture in an undifferentiated state (Figures 1, 3, and 6). Near confluence, most of the cells have morphological features of undifferentiated cells, and express characteristic phenotypic markers: SSEA-4, Tra-1-60, Tra-1-81, Oct-4, Cripto, and telomerase reverse transcriptase (TERT). The cultures retain their pluripotency, demonstrated by their ability to form differentiated cells representing all three germ layers.

It has been discovered that an unusually high concentration of FGF in a suitable base nutrient medium is often adequate to maintain human embryonic stem (hES) cells in a substantially undifferentiated form. This is counter-intuitive, because FGF is thought to drive cells *towards the formation of fibroblasts.* There has been no previous suggestion to increase FGF concentrations in the culture medium for any cell type, once serum has been removed from the culture.

Quite surprisingly, it has now been found that hES cells grown in high FGF containing medium with glutamine and bicarbonate expand substantially more rapidly than hES cells grown on feeder cells or in conditioned medium, *while maintaining their undifferentiated phenotype.* The reasons for this are unclear; nor was it predictable based on what was previously known about hES cell culture. Nevertheless, this finding is important, because it provides a rapid expansion method for producing commercial grade undifferentiated hES cells on a commercial scale. Now that this technology is available, the production of pluripotent stem cells for treating human patients in need of tissue regeneration holds considerable promise.

The techniques provided in this invention represent an important advance in the potential use of pluripotent stem cells for research and therapeutic use. Further advantages of the invention will be understood from the sections that follow.

### Definitions

Prototype "human Embryonic Stem cells" (hES cells) are described by Thomson et al. (Science 282:1145, 1998; U.S. Patent 6,200,806). Those skilled in the art will appreciate that the term includes established lines that bear phenotypic characteristics of hES cells, and derivatives of such lines that still have the capacity of producing progeny of each of the three germ layers.

hES cell cultures are described as "undifferentiated" when a substantial proportion of stem cells and their derivatives in the population display morphological characteristics of undifferentiated cells, clearly distinguishing them from differentiated cells of embryo or adult origin. Undifferentiated hES cells are easily recognized by those skilled in the art, and typically appear in the two dimensions of a microscopic view with high nuclear/cytoplasmic ratios and prominent nucleoli. It is understood that colonies of undifferentiated cells within the population will often be surrounded by neighboring cells that are differentiated. Nevertheless, the undifferentiated colonies persist when the population is cultured or passaged under appropriate conditions, and individual undifferentiated cells constitute a substantial proportion of the cell population. Cultures that are substantially undifferentiated contain at least 20% undifferentiated hES cells on an ongoing basis, and may contain at least 40%, 60%, or 80% in order of increasing preference (in terms percentage of cells with the same genotype that are undifferentiated).

Whenever a culture or cell population is referred to In this disclosure as proliferating "without differentiation", what is meant is that after proliferation, the composition is substantially undifferentiated according to the preceding definition. Populations that proliferate through at least four passages (~20 doublings) without differentiation will contain substantially the same proportion of undifferentiated cells (or possibly a higher proportion of undifferentiated cells) when evaluated at the same degree of confluence as the originating culture.

"Feeder cells" or "feeders" are terms used to describe cells that can be co-cultured with pPS cells to allow them to proliferate without differentiation. pPS cell populations are said to be "essentially free" of feeder cells if the cells have been grown through at least one round after splitting in which fresh feeder cells are not added to support the growth of pPS cells. A feeder free culture will contain less than about ~5% feeder cells. Compositions containing less than 1%, 0.2%, 0.05%, or 0.01% feeder cells (expressed as % of total cells in the culture) are increasingly more preferred.

A "growth environment" is an environment in which cells of interest will proliferate in vitro. Features of the environment include the medium in which the cells are cultured, and a supporting structure (such as a substrate on a solid surface) if present.

A "nutrient medium" is a medium for culturing cells containing nutrients that promote proliferation. The nutrient medium typically contains isotonic saline, buffer, a protein source (in the form of one or more added proteins or amino acids), and potentially other exogenously added nutrients and growth factors.

A "conditioned medium" is prepared by culturing a first population of cells in a medium, and then harvesting the medium. The conditioned medium (along with anything secreted into the medium by the cells) may then be used to support the growth of a second population of cells. Where a particular ingredient or factor is described as having been added to the medium, what is meant is that the factor (or a cell or particle engineered to secrete the factor) has been mixed into the medium by deliberate manipulation.

A "fresh medium" is a medium that has not been purposely conditioned by culturing with a different cell type before being used with the cell type it is ultimately designed to support. Otherwise, no limitations are intended as to its manner of preparation, storage, or use. It is added fresh (by exchange or infusion) Into the ultimate culture, where it may be consumed or otherwise processed by the cell types that are present.

A "commercial product" is any product or product combination that is manufactured for distribution from one entity to another entity for any purpose, with or without financial compensation or other consideration from the other entity. Exemplary is a culture medium or culture environment, with or without other reagents, containers, or apparatus for use in culturing cells, and with or without written Information.

### General Techniques

General methods in molecular genetics and genetic engineering are described in the current editions of Molecular Cloning: A Laboratory Manual, (Sambrook et al., Cold Spring Harbor); Gene Transfer Vectors for Mammalian Cells (Miller & Calos eds.); and Current Protocols in Molecular Biology (F.M. Ausubel et al. eds., Wiley & Sons). Cell biology, protein chemistry, and antibody techniques can be found in Current Protocols in Protein Science (J.E. Colligan et al. eds., Wiley & Sons); Current Protocols in Cell Biology (J.S. Bonifacino et al., Wiley & Sons) and Current Protocols in Immunology (J.E. Colligan et al. eds., Wiley & Sons.). Reagents, cloning vectors, and kits for genetic manipulation referred to in this disclosure are available from commercial vendors such as BioRad, Stratagene, Invitrogen, ClonTech, and Sigma-Aldrich Co.

Cell culture methods are described generally in the current edition of Culture of Animal Cells: A Manual of Basic Technique (R.I. Freshney ed., Wiley & Sons); General Techniques of Cell Culture (M.A. Harrison & I.F. Rae, Cambridge Univ. Press), and Embryonic Stem Cells: Methods and Protocols (K. Turksen ed., Humana Press). Other texts are Creating a High Performance Culture (Aroselli, Hu. Res. Dev. Pr. 1996) and Limits to Growth (D.H. Meadows et al., Universe Publ. 1974). Tissue culture supplies and reagents are available from commercial vendors such as Gibco/BRL, Nalgene-Nunc International, Sigma Chemical Co., and ICN Biomedicals.

### Sources of pluripotent stem cells

Suitable source of hES cells for culturing and differentiation according to this disclosure are established lines.

### Propagation of hES cells in the absence of feeder cells

The medium of this invention allows hES to be propagated in an undifferentiated state, even in the absence of feeder cells. Feeder-free hES cell cultures can be obtained either by passaging cells grown on feeder into feeder-free conditions, or by first deriving the cells from blastocysts into a feeder-free environment.

In the absence of feeders, the hES cells are cultured in an environment that supports proliferation without differentiation. Aspects of culture that can affect differentiation include the substrate upon which the cells are cultured, the medium in which they are cultured, and the manner in which they are split and passaged to new culture environments.

hES cells can be supported in feeder-free culture on an extracellular matrix. The matrix can be deposited by preculturing and lysing a matrix-forming cell line (U.S. 6,800,480), such as the STO mouse fibroblast line (ATCC Accession No. CRL-1503), or human placental fibroblasts. The matrix can also be coated directly into the culture vessel with isolated matrix components. Matrigel is a soluble preparation from Engelbreth-Holm-Swarm tumor cells that gels at room temperature to form a reconstituted basement membrane. Other suitable extracellular matrix components may include laminin, fibronectin, proteoglycan, vitronectin, entactin, heparan sulfate, and so on, alone or in various combinations. The matrix components may be human, and/or produced by recombinant expression. Substrates that can be tested using the experimental procedures described herein include not only other extracellular matrix components, but also polyamines, and other commercially available coatings. This invention contemplates adding extracellular matrix to the fluid phase of a culture at the time of passaging the cells or as part of a regular feeding. This invention also contemplates extracellular matrix deposited into the culture by cells within the culture (such as stromal cells that have formed from the pPS cell line around the periphery of an undifferentiated colony).

The hES cells are plated onto the substrate in a suitable distribution and in the presence of a medium that promotes cell survival, propagation, and retention of the desirable characteristics. These characteristics benefit from careful attention to the seeding distribution. Plating densities of at least -15,000 cells cm⁻² (typically 90,000 cm⁻² to 170,000 cm⁻²) promote survival and limit differentiation. In one method, enzymatic digestion is halted before cells become completely dispersed (say, ~5 min with collagenase IV). The plate is then scraped gently with a pipette, and the cells are triturated into clumps of adherent cells, about 10-2000 cells in size, which are then passaged into the new culture environment.

Alternatively, hES cells can be passaged between feeder-free cultures as a finer cell suspension, providing that an appropriate enzyme and medium are chosen, and the plating density is sufficiently high. By way of illustration, confluent human embryonic stem cells cultured in the absence of feeders are removed from the plates by incubating with 0.05% (wt/vol) trypsin and 0.053 mM EDTA for 5-15 min at 37°C. The remaining cells in the plate are removed, triturated with the pipette until dispersed into single cells and small clusters, and then replated. In another illustration, the cells are harvested without enzymes before the plate reaches confluence. The cells are incubated ~5 min in 0.5 mM EDTA alone in PBS, washed from the culture vessel, and then replated without further dispersal.

hES cells plated in the absence of fresh feeder cells benefit from being cultured in a nutrient medium of the invention as defined in the claims. The medium will contain the usual components to enhance cell survival, including isotonic buffer (i.e., a buffer that is isotonic when adjusted to working concentration), essential minerals, and either serum or a serum replacement of some kind. To inhibit differentiation, the medium is formulated to supply some of the elements provided by feeder cells or their equivalents.

The base nutrient medium used can have any of several different formulae. Exemplary serum-containing ES medium is made with 80% DMEM (typically KO DMEM), 20% defined fetal bovine serum (FBS), 1% non-essential amino acids, 1 mM L-glutamine, and 0.1 mM β-mercaptoethanol. Serum-free ES medium is made with 80% KO DMEM, 20% serum replacement (SR: Gibco #10828-028), 1% non-essential amino acids, 1 mM L-glutamine, and 0.1 mM β-mercaptoethanol.

### Medium additives

The nutrient medium of the invention used for culturing the hES cells comprises one or more factors that promote proliferation of the pPS cells without differentiation as defined in the claims. As will be apparent from the following description, the supplementation can occur by preculturing the medium with cells that secrete such factors, by adding such factors to the medium artificially, or by both techniques in combination.

Conditioned medium can be prepared by culturing irradiated primary mouse embryonic fibroblasts or other feeder cells at a density of -5-6 x 10⁴ cm⁻² in a serum free medium such as KO DMEM supplemented with 20% serum replacement and -4-8 ng/mL basic fibroblast growth factor (bFGF). The culture supernatant is harvested after -1 day at 37°C. The cells are cultured in the medium for sufficient time to allow adequate concentration of released factors that support pPS cell culture. Typically, medium conditioned by culturing for 24 hours at 37°C contains a concentration of factors that support pPS cell culture for at least 24 hours. However, the culturing period can be adjusted upwards or downwards, determining empirically what constitutes an adequate period. Medium that has been conditioned for 1-2 days Is typically used to support pPS cell culture for 1-2 days, and then exchanged.

Non-conditioned medium that supports pPS cell growth in an undifferentiated state can be created by adding to a suitable base medium certain factors that invoke the appropriate signal transduction pathways in undifferentiated cells.

Suitable base media include ES cell medium with 20% serum replacement, as already described. Other base media that can be used for this purpose are commercially available for culturing proliferative cell types like hematopoietic cells. Exemplary are X-VIVO 10 expansion medium (Biowhittaker) and QBSF™-60 (Quality Biological Inc.) (Example 4). See also WO 98/30679 (Life Technologies Inc.) and U.S. 5,405,772 (Amgen). The medium will typically contain a neutral buffer (such as phosphate and/or high concentration bicarbonate) in isotonic solution; and protein nutrient, in the form of serum (such as FBS), serum replacement (SR), albumin (e.g., isolated or recombinant human albumin), or essential and non-essential amino acids (such as glutamine) in an effective combination. It will also typically contain lipids, fatty acids, or cholesterol as artificial additives or the HDL or LDL extract of serum. Other beneficial factors that can be Included are growth-inducing hormones like insulin or transferrin, nucleosides or nucleotides, pyruvate, a sugar source (such as glucose), selenium (in any ionized form or salt), a glucocorticoid (such as hydrocortisone) or other substance that inhibits activation of Inflammatory pathways, or a reducing agent (such as β-mercaptoethanol).

It has been discovered that adding a fibroblast growth factor at high concentration of at least 40ng/ml is especially effective to promote hES cell proliferation without differentiation. Exemplary are basic FGF (FGF-2), and FGF-4, but other members of the family can also be used. Equivalent forms as species homologs, artificial analogs, antibodies to the respective FGF receptor, and other receptor activating molecules. It has been determined from gene expression analysis that undifferentiated hES cells express receptors for acidic FGF (FGF-1). At a high concentration, FGF alone is sufficient to promote growth of hES cells in an undifferentiated state (Examples 5 and 6).

As a supplement to FGF, ligands that bind c-kit, such as stem cell factor (SCF, Steel factor), antibodies to c-kit, and other activators of the same signal transduction pathway may also be beneficial. SCF is dimeric and occurs in soluble and membrane-bound forms. It transduces signals by ligand-mediated dimerization of c-kit, which is a receptor tyrosine kinase related to the receptors for platelet-derived growth factor (PDGF), macrophage colony-stimulating factor, Flt-3 ligand and vascular endothelial growth factor (VEGF). Addition of 15 ng/mL Flt-3 ligand or SCF to medium containing 40 ng/mL bFGF improves culture characteristics - in fact, 15 ng/mL Flt-3 ligand plus 40 ng/mL FGF is about as effective as 80 ng/mL bFGF alone (Examples 5 and 6). Other possible additives are factors that elevate cyclic AMP levels, such as forskolin; factors that induce gp130, such as LIF or Oncostatin-M; and hematopoietic growth factors, such as thrombopoietin (TPO) (Example 3) Such factors or their equivalents may be used individually or in an effective combination with other influential factors in the medium, as already described.

The culture media is also supplemented with TGFβ1, for example, TGFβ2 or TGFβ3, transforming growth factor β1 (TGFβ1) (M. Amit et al., Biol. Reprod. 70:837, 2004) (also within the concentration ranges provided in this paragraph). The culture media may be supplemented with between 0.01 ng/ml and 10 ng/ml TGFβ1; or between 0.1 ng/ml and 5 ng/ml TGFβ1; or between 0.2 ng/ml and 1.0 ng/ml TGFβ1; or between 0.4 ng/ml and 0.6 ng/ml TGFβ1; or with at least 0.5 ng/ml TGFβ1. For example, the culture media may include 80 ng/ml hbFGF and 0.5 ng/ml FGFβ1 (see Example 7).

Whenever these media are built from scratch, it is possible to use extracts of human serum, human recombinant proteins, and biosynthetic components in any suitable combination, thereby avoiding the use of any component originating from non-human vertebrate animals. The media may be "defined", which means that undefined mixtures or extracts (e.g., protein or lipid blends obtained from serum or other sources) are avoided in favor of purified components of known composition. Each of these properties has advantages in terms of the reproducibility and reliability of the formulation, and satisfaction of regulatory issues relating to the use of pPS derived products in human clinical therapy. The skilled reader will understand from this description the particular suitability of deriving and/or culturing pPS cell lines in an environment comprising only human and synthetic components. The culture medium, and the extracellular matrix or feeder cells, if used, can all be assembled with human biological products and recombinantly produced equivalents, thereby avoiding the use of any non-human animal products throughout the process.

### Desirable outcomes

A medium formulation can be tested for its ability to support hES cells by swapping it into a feeder-free culture system in place of medium conditioned by primary mouse embryonic fibroblasts (mEF), or some other proven standard (Examples 3, 5, and 6). If hES cells grow in a substantially undifferentiated state, then the medium can be characterized as supporting hES cells in feeder free culture.

One of the virtues of using fresh medium in this culture system is the ability to adjust conditions so that the cells expand more rapidly than they do when cultured on feeder cells according to traditional techniques, or in conditioned medium. Populations of pluripotent stem cells can be obtained that are 10-, 20-, 50-, 100-, or 1000-fold expanded when compared to the starting population. Under suitable conditions, cells in the expanded population will be 50%, 70% or more in the undifferentiated state.

The degree of expansion per passage is calculated by dividing the number of cells harvested at the end of the culture by the number of cells originally seeded into the culture. Where geometry of the culture environment is limiting or for other reasons, the cells may optionally be passaged into a similar culture environment for further expansion. The total expansion is the product of all the expansions in each of the passages. Of course, it is not necessary to retain all the expanded cells on each passage. For example, if the cells expand 2-fold in each culture, but only ~50% of the cells are retained on each passage, then approximately the same number of cells will be carried forward. But after four cultures, the cells are said to have undergone an expansion of 16-fold.

Cultures of hES cells on mouse embryonic fibroblast (mEF) feeder cells, or in mEF conditioned medium, have a doubling time of about 31-33 hours (Example 1). Certain culture environments of this invention comprising fresh medium support doubling of hES cells in less than -24 hours (Example 5), potentially in less than -16 hours. In terms of expansion upon regular passaging in standard culture wells, the system can be used to expand hES cells by 10- to potentially 50-fold per week. Improved efficiency is believed to be the result both of the more rapid doubling time, and the higher proportion of pPS cells that take in the new environment after passaging.

Of course, culture conditions inappropriate for hES cells will cause them to differentiate promptly. However, the reader should be aware that marginally beneficial conditions may allow hES cells to go through a few passages while still retaining a proportion of undifferentiated cells. In order to test whether conditions are adequate for indefinite culture of hES cells, it is recommended that the cells be expanded at least 10- or 20-fold though at least four passages. A higher degree of expansion and/or a higher number of passages (e.g., at least 7 passages and 50- or 100-fold expansion) provides a more rigorous test. It is permissible for a few phenotypic markers to undertake a quantitative adjustment befitting adaptation to particular conditions (say, up or down 2- or 5-fold) - they will typically revert to previous levels when the cells are placed back into their previous environment (Example 5).

An effective test for whether a cell is still pluripotent is the demonstration that it can still be caused to differentiate into progeny that represents (or bears antibody or PCR-detectable phenotypes) of each of the three embryonic germ layers. As a proxy, the user may identify the undifferentiated phenotype using the marker system described in the next section.

Nutrient medium and other culture characteristics formulated according to this invention can be adapted to any culture device suitable for growing hES cells. Devices having a suitable surface include regular tissue culture wells, T-flasks, roller bottles, gas-permeable containers, and flat or parallel plate bioreactors or cell factories. Also contemplated are culture environments in which the hES cells are attached to microcarriers or particles kept in suspension in stirred tank vessels. Fresh medium can be introduced into any of these environments by batch exchange (replacement of spent medium with fresh medium), fed-batch process (fresh medium added with no removal), or ongoing exchange in which a proportion of the medium is replaced with fresh medium on a continuous or periodic basis.

### Characteristics of undifferentiated hES cells

Human ES cells have the characteristic morphological features of undifferentiated stem cells. In the two dimensions of a standard microscopic image, hES cells have high nuclear/cytoplasmic ratios in the plane of the image, prominent nucleoli, and compact colony formation with poorly discernable cell junctions. Cell lines can be karyotyped using a standard G-banding technique (available at many clinical diagnostics labs that provides routine karyotyping services, such as the Cytogenetics Lab at Oakland CA) and compared to published human karyotypes. It is desirable to obtain cells that have a "normal karyotype", which means that the cells are euploid, wherein all human chromosomes are present and are not noticeably altered.

When cells are split and passaged between cultures, some cells may differentiate (particularly when replated as single cells at low density, or when large clusters are allowed to form). However, cultures typically reestablish a larger proportion of undifferentiated cells as they reapproach confluence.

hES and hEG cells can also be characterized by expressed cell markers detectable by antibody (flow cytometry or immunocytochemistry) or by reverse transcriptase PCR. Human ES cells typically have antibody-detectable SSEA-4, Tra-1-60, and Tra-1-81, but little SSEA-1. hES cells can also be characterized by the presence of alkaline phosphatase activity, which can be detected by fixing the cells with 4% paraformaldehyde, and then developing with Vector Red as a substrate, as described by the manufacturer (Vector Laboratories, Burlingame CA).

Cultures can also be characterized according to gene expression determined at the mRNA level. Panels of suitable markers are listed in application US 2003/0224411 A1 (Geron Corp.) Exemplary are Cripto, gastrin-releasing peptide (GRP) receptor, podocalyxin-like protein (PODXL), human telomerase reverse transcriptase (hTERT), and the POU transcription factor Oct 3/4. Telomerase enzyme activity (characteristic of hES cells) can be measured by TRAP assay (Example 6). The cultures can also be back-screened against markers of differentiated cells, which should be low if the culture system is promoting proliferation without differentiation. Such markers may include CD44, CD105 (endoglin), CD106(VCAM-1), CD90 (Thy-1), STRO-1, Vimentin, and Human Thymus Stroma.

Another desirable feature of propagated hES cells is a potential to differentiate into cells of all three germ layers: endoderm, mesoderm, and ectoderm. Pluripotency of hES cells can be confirmed by forming teratomas in SCID mice, and examining them for representative tissues of all three germ layers. Alternatively, pluripotency can be determined by allowing hES cells to differentiate non-specifically (for example, by forming embryoid bodies), and then determining the cell types represented in the culture by immunocytochemistry (Figure 6).

### Commercial distribution

The tissue culture media as described herein is distributed for use in maintaining or expanding hES cells in a substantially undifferentiated form, with or without subsequent differentiation into a particular mature cell type. The media can be constituted in any workable formulation, such as:
- media ready for culture;
- concentrated media to be diluted prior to use in culture;
- powdered media (or any equivalent solid form) to be dissolved in water or an aqueous solvent prior to use in culture;
- various components of the media (in solution, and/or in solid form) to be combined before use in culture.

Of course, such formulation options may be used in combination. For example, a media kit may comprise a base medium to be diluted to isotonic strength, plus concentrated and/or solid nutrients and growth factors to be combined into the medium to make up the desired medium constitution. Any reagent or set of reagents for commercial sale that makes up the media of this invention when properly assembled should be regarded as equivalent to the media per se, unless indicated otherwise.

Optionally, extracellular matrix can be distributed in combination with the media: either in the same packaging, or as complementary components catalogued as being useful in a combined culture system. Optionally, the combination can also include a tissue culture device, or other equipment or reagents useful for culturing or characterizing the cells.

The products and product combinations are packaged in suitable containers, optionally in kit form, and may be accompanied by written information on the use of the materials according to this invention - such as maintaining or expanding pPS cells. The information may be written in any language on any communication medium accessible and understandable by the intended user. It may take the form of a label on the container or the kit, or a product insert packaged with the container and distributed together. Equivalent forms are descriptions, instructions, or explanations written in hard copy or in electronic form available to the user or the intended user as reference or resource material associated with the commercially distributed product.

*The examples that follow are illustrations not meant to limit the claimed invention*

### REFERENCE EXAMPLES

### Example 1: Growing hES cells without feeder cells in conditioned medium

In this example, undifferentiated hES cells that had been maintained on primary mouse embryonic feeder cells were maintained in the absence of feeders. The culture wells were coated with Matrigel, and the cells were cultured in the presence of conditioned nutrient medium obtained from a culture of irradiated primary fibroblasts.

Conditioned medium (CM) was prepared as follows. The fibroblasts were harvested from T150 flasks by washing once with Ca⁺⁺/Mg⁺⁺ free PBS and incubating in trypsin/EDTA (Gibco). After the fibroblasts detached from the flask, they were collected in mEF medium (DMEM + 10% FBS). The cells were irradiated at 4000 rad, counted and seeded at about 55,000 cells cm⁻² in mEF medium. After at least 4 hours, the medium was exchanged with SR containing ES medium. Conditioned medium was collected daily for feeding of hES cultures. Alternatively, medium was prepared using mEF plated in culture flasks, exchanging medium daily at 0.3-0.4 mL cm⁻². Before addition to the hES cultures, the conditioned medium was supplemented with 4 ng/mL of human bFGF (Gibco). Fibroblast cultures were used in this system for about 1 week, before replacing with newly prepared cells.

Undifferentiated hES colonies were harvested from hES cultures on feeders as follows. Cultures were incubated in ~200 U/mL collagenase IV for about 5 minutes at 37 °C. Colonies were harvested by picking individual colonies up with a 20 µL pipet tip under a microscope or by scraping and dissociating into small clusters. These cells were then seeded onto Matrigel coated plates in conditioned medium (CM).

The day after seeding on Matrigel, hES cells were visible as small colonies and there were cells in between the colonies that appeared to be differentiating or dying. As the hES cells proliferated, the colonies became quite large and very compact, representing the majority of surface area of the culture dish. The hES cells in the colonies had a high nucleus to cytoplasm ratio and had prominent nucleoli, similar to hES cells maintained on feeder cells. At confluence, the differentiated cells in between the colonies represented less than 10% of the cells in the culture.

Six days after seeding, the cultures had become almost confluent. The cultures were split using Collagenase IV, gently triturated into small clusters of 10-2,000 cells, and then re-seeded on Matrigel coated plates in conditioned medium at ~90,000 to 170,000 cells cm⁻². Medium was changed daily, and the cells were split and passaged again at 13 and 19 days after initial seeding.

Cultures of hES cells have been grown in the absence of feeder cells for over one year, with no apparent change in the proliferative capacity or phenotype. Human ES cells maintained on Matrigel in mEF conditioned medium have a doubling time of about 31-33 hours, similar to the proliferation rate for hES cells grown on mEF feeder cells. H1 cells after 64 days of feeder-free culture showed a normal karyotype.

hES cells seeded onto laminin, fibronectin or collagen IV had colonies of undifferentiated hES cells, although the cultures on fibronectin or collagen IV did not contain as many undifferentiated colonies as the cultures on Matrigel or laminin. When cells on Matrigel or laminin reached confluence, the cells within the colonies became very compact, were morphologically very similar to the cells maintained on feeders and were serially passaged. After 40 days (6 passages), cells on Matrigel and laminin contained a high proportion of colonies which continued to display ES-like morphology in long-term culture. However, cells maintained on fibronectin or collagen IV had fewer colonies displaying appropriate ES-morphology. As controls, cells cultured on Matrigel or laminin in non-conditioned medium appeared to be proliferating more slowly and showed a differentiated morphology after a few passages.

Human ES cells maintained on Matrigel in mEF conditioned medium showed a doubling time of about 31-33 hours, similar to the proliferation rate for hES cells grown on mEF feeder cells. Long-term culture in feeder free conditions can maintain a normal karyotype (Rosler et al., Dev Dyn. 229:259, 2004; Carpenter et al., Dev Dyn. 229:243, 2004).

### Example 2: Characterization of hES cells grown in conditioned medium

Undifferentiated hES cells express SSEA-4, Tra-1-60, Tra-1-81, OCT-4, and hTERT. In order to assess whether the cells maintained in feeder-free conditions retained these markers, cells were evaluated by immunostaining, reverse transcriptase PCR amplification, and assay for telomerase activity.

For analysis by fluorescence-activated cell sorting (FACS), the hES cells were dissociated in 0.5 mM EDTA in PBS and resuspended to about 5 x 10⁵ cells in 50 µL diluent containing 0.1% BSA in PBS. They were labeled with specific primary antibody and then fluorescent second antibody, and analyzed on a Flow Cytometer.

Similar to the hES cells on feeders, cells on Matrigel, laminin, fibronectin or collagen IV expressed SSEA-4, Tra-1-60 and Tra-1-81. There was very little expression of SSEA-1, a glycolipid that is not expressed by undifferentiated hES cells. SSEA-4, Tra-1-60, Tra-1-81, and alkaline phosphatase were expressed by the hES colonies on Matrigel or laminin, as seen for the cells on feeders - but not by the differentiated cells in between the colonies.

Telomerase activity was measured by telomeric repeat amplification protocol (TRAP assay: Kim et al., Science 266:2011, 1997; Weinrich et al., Nature Genetics 17:498, 1997). All the cultures conditions showed positive telomerase activity after 40 days on Matrigel, laminin, fibronectin or collagen IV in mEF conditioned medium.

hES cells were also tested for their ability to form teratomas by intramuscular injection into SCID mice. Cells maintained on feeders or off feeders were harvested, resuspended in PBS and injected intramuscularly into SCID/beige mice (5 x 10⁶ cells per site). Tumors were excised and processed for histological analysis. Cystic epithelial structures, probable dental component, cartilage and glandular epithelial or neural components were found in teratomas derived from feeder-free hES cultures.

### Example 3: Additives that promote undifferentiated ES cell growth in fresh medium

- Experiments were conducted to investigate how different growth factors influence the proliferation and maintenance of undifferentiated hES cells of the H9 cell line.

hES medium contained 20% Serum Replacement (Gibco #10828-028), 80% Knockout DMEM (Gibco #10829-018), 1% non-essential amino acids (Gibco #11140-050), 1 mM L-glutamine (Gibco #15039-027), and 2.5 mM β-mercaptoethanol (Sigma #M7522). This medium was supplemented with 40 ng/mL bFGF; 15 ng/mL stem cell factor (SCF, R&D System #255SC); 100 ng/mL leukemia inhibitory factor (LIF, Sigma #L5283 or Chemicon #LIF 1010); 50 ng/mL ciliary neurotrophic factor (CNTF, R&D system #257-NT); 50 ng/mL recombinant human Oncostatin M (OSM, Sigma #O9635); and 15 ng/mL interleukin 6 (IL-6, R&D System #206-IL).

The H9 cell line (passage 31) was harvested from a culture in conditioned medium, plated onto Matrigel, and cultured with hES medium with the factors at the concentrations indicated above, or 5- or 25-fold lower. Cells grown in the fully supplemented medium displayed undifferentiated hES morphology. A higher degree of differentiation was observed after four passages for the cultures grown at lower concentrations of the growth factors, and the cells maintained without growth factors were almost completely differentiated. These cultures were terminated.

After six passages, cells from the full-strength cocktail were replated onto Matrigel as before, or onto laminin, which is free of the growth factors contained in the Matrigel matrix. After eight passages, a large percentage of cells (~50-70%) in cultures grown on Matrigel or laminin in this medium continued to display undifferentiated hES morphology. Some cells on Matrigel or laminin were then passaged into hES medium containing added 40 ng/mL bFGF; but not SCF, LIF, CNTF, OSM, or IL-6. The cells continued to show an undifferentiated phenotype for the next four passages.

As detected by immunocytochemistry, expression patterns and levels of surface markers, including SSEA-1, SSEA-4, Tra 1-60 and Tra 1-81 in cultures maintained in high concentrations of growth factors were similar to cells maintained in MEF conditioned medium (MEF-CM). Cell lines stained positively for histocompatibility Class I antigen (HLA-ABC), and were negative in the isotype control (msIgG1). 50-70% of cells in cultures maintained in growth factors or MEF-CM expressed c-kit (a receptor for stem cell factor) while less than 20% of cells expressed gp130 (associated with the LIF receptor). This pattern supports the hypothesis that ligands for c-kit help support undifferentiated hES cell growth.

After 14 passages in the full-strength growth factor cocktail (~70 population doublings), about 50-70% of cells cultured on Matrigel or laminin displayed morphology of undifferentiated hES cells, and had a normal karyotype. Cells cultured without any growth factor showed almost complete differentiation after 4 weeks in culture. A high degree of differentiation was also observed for cultures in which the growth factors had been diluted by 5- or 25-fold.

Additional experiments were done to dissect the components in the factor cocktail essential for hES cell growth. Cells of the H7 line were cultured in fresh hES medium containing the factor combinations shown in Table 2.

| | Table 2: Factors added to fresh ES medium for hES cell culture | |
|---|---|---|
| Condition | bFGF | Other Growth Factors |
| A | 8 ng/mL | |
| B | | |
| C | 40 ng/mL | |
| D | 40 ng/mL | SCF (15 ng/mL) |
| E | 40 ng/mL | Flt-3L (75 ng/mL) |
| F | 40 ng/mL | TPO (100 ng/mL) |
| G | 40 ng/mL | LIF (100 ng/mL) |
| H | 40 ng/mL | SCF (15 ng/mL), IL-6 (15 ng/mL), LIF (100 ng/mL), CNTF (50 ng/mL), OSM (50 ng/mL) |
| I | | SCF (15 ng/mL) |
| J | | SCF (100 ng/mL) |
| K | | Flt-3L (75 ng/mL) |
| L | | TPO (100 ng/mL) |
| M | | SCF (15 ng/mL), Flt-3L (75 ng/mL) |
| N | | SCF (15 ng/mL), TPO (100 ng/mL) |
| O | | SCF (100 ng/mL), Flt-3L (100 ng/mL), IL-6 (15 ng/mL) |
| P | 40 ng/mL | SCF (15 ng/mL), Flt-3L (75 ng/mL) |
| Q | 40 ng/mL | SCF (15 ng/mL), TPO (100 ng/mL) |

Cultures were passaged in these conditions and evaluated on an ongoing basis by morphological criteria. Many of the conditions continued to maintain considerable numbers of undifferentiated colonies.

**Figure 1** shows morphology of hES cells maintained for 6 passages in conditioned medium (mEF-CM), fresh SR medium alone, or fresh medium supplemented with 40 ng/mL bFGF or bFGF with stem cell factor. bar = 800 µm.

**Figure 2** shows immunocytometric analysis for expression of SSEA-4 and Tra-1-81. Conditions A, C, D, and E all maintained SSEA-4 and Tra-1-81 expression in a large proportion of the H7 cells. A subpopulation expressing these markers at high levels is also shown. Percentage of cells that show high-level staining for both TRA-1-81 and SSEA4 is indicated on the right side.

**Figure 3** compares SSEA-4 and Tra-1-81 staining amongst different growth conditions afar 6 passages. The presence of 40 ng/mL FGF (marked by "+") showed an undifferentiated phenotype comparable to cells grown in mEF conditioned medium; whereas combinations of growth factors without FGF (marked by"-") showed partial differentiation. These findings were confirmed using the H9 cell line. Undifferentiated colonies of H9 cells maintained in fresh medium containing bFGF at 40 ng/mL for 15 passages expressed SSEA-4, Tra-1-61, Tra-1-81 and alkaline phosphatase.

OCT3/4, hTERT, and cripto are markers for identifying the undifferentiated hES cell phenotype at the mRNA level (US-2003-0224411-A1). Quantitative real-time PCR assays were conducted to determine the expression of these markers in H7 cells after 6 passages. Cells cultured in bFGF-containing medium maintained expression of the markers at levels comparable to cells in mEF conditioned medium.

**Figure 4** shows telomerase activity and karyotype analysis of cells maintained in the factors listed in Table 2. Consistent with the expression of hTERT, hES cells maintained in bFGF alone or with other factors for 15 passages had telomerase activity as measured by TRAP assay. Cytogenetic analysis was performed using H7 cells maintained in MEF-CM, bFGF alone or with other factors (condition A, C, D, E, F, P and Q) for 15 passages. Cultures maintained a normal karyotype in all conditions except cultures in condition Q, where 4 of 30 metaphases were abnormal (trisomy 12). Three independent H9 cultures maintained in bFGF alone at passage 4 and 15 and one H9 cell culture maintained in bFGF + SCF + IL-6 (condition H) at passage 14 was also found to have a normal karyotype.

**Figure 5** shows that H7 hES cells maintained in bFGF alone or in combination with other factors through 15 passage retained their pluripotency. They readily formed embryoid bodies that were further differentiated for 7 days. Immunocytochemical analysis demonstrated cells staining positively for β-tubulin III, α-fetoprotein (AFP), or smooth muscle actin (Panel A). The hES cells also retained the capacity to generate teratomas when injected to SCID/beige mice (Panel B), like the cells grown in mEF conditioned medium. Histological analysis indicated the presence of multiple tissue structures including cartilage, primitive renal tissue, glandular epithelium, pigmented epithelium, nervous tissue and mesenchymal tissue. Therefore, cells grown in fresh medium containing high bFGF concentration retained their ability to differentiate into cells representing all three germ layers.

### Example 4: Media for growing hES cells with added FGF

hES cells passaged 29 times in conditioned medium were weaned onto an alternative medium designed for proliferation and development of hematopoietic cells.

Ex vivo expansion medium was obtained by arrangement with a commercial supplier, and is thought to be based on the medium described in U.S. Patent 5,405,772 (Ponting, Amgen Inc.). The Ponting medium comprises the following components: Iscove's modified Dulbecco's medium; amino acids; vitamins, bovine albumin; bovine transferrin (100 µg/mL); lipids and cholesterol; β-mercaptoethanol; pyruvate; nucleotides; epidermal growth factor (15 ng/mL); fibroblast growth factor (2ng/mL); platelet-derived growth factor (10ng/mL); and insulin (10 µg/mL). For use in the current experiments, the medium was further supplemented with 2 mM L-glutamine, 1% non-essential amino acids (Gibco), 0.1 mM β-mercaptoethanol, and 8 ng/mL bFGF.

The cells were first passaged onto Matrigel coated plates using collagenase IV, and cultured for 2 days with conditioned medium. On day 2, the 100% conditioned medium was replaced with medium containing 80% conditioned medium plus 20% fresh expansion medium. Cells were fed fresh daily and passaged weekly. The proportion of expansion medium was increased by 20% approximately every 2 days until the cells were completely weaned, and then grown until they had been passaged a further 8 times.

At passages 1-4 in the expansion medium, the proportion of cells with the morphology of undifferentiated phenotype appeared to diminish slightly, but was restored by passage 8. When these cells were passaged back to medium conditioned by primary mouse embryonic fibroblasts, the cells were indistinguishable from those grown throughout the period in conditioned medium by the second passage.

To confirm that these cells retained their pluripotency, embryoid bodies were formed and analyzed by immunocytochemistry for phenotypic markers representing each of the three germ layers. After passage 4 in expansion medium, the cells were dissociated into small clumps using 200 U/mL collagenase IV at 37°C for 10 min placed in suspension culture in differentiation medium (DMEM + 10% or 20% FBS) for 4 days, then transferred onto poly-L-ornithine hydrobromide coated plates and cultured a further 10 days. They were fixed in 4% paraformaldehyde, permeabilized, and labeled alternately with mouse anti human β-tubulin isotype III clone SDL.3D10, mouse anti human muscle actin clone HHF35, or mouse anti α-fetoprotein. Primary antibody was visualized using FITC labeled goat anti-mouse IgG. Results showed that hES cells passaged repeatedly in expansion medium (not previously conditioned), and then differentiated, were positive for β-tubulin and muscle actin.

### Example 5: Rapid expansion method for producing pluripotent stem cells

hES cells passaged 20 times in conditioned medium were weaned onto an alternative medium designed for proliferation of human hematopoietic cells. X-VIVO 10 expansion medium was obtained from Biowhittaker; QBSF™-60 was obtained from Quality Biological Inc. The X-VIVO 10 formulation contains pharmaceutical grade human albumin, recombinant human insulin and pasteurized human transferrin. Exogenous growth factors, artificial stimulators of cellular proliferation or undefined supplements are not included in the X-VIVO 10 medium. They are also devoid of any protein-kinase C stimulators. QBSF™-60 is a serum-free formulation that contains recombinant or pasteurized human proteins. For use in these experiments, the X-VIVO 10 medium was supplemented with 2 mM L-glutamine, 1% non-essential amino acids (Gibco), 0.1 mM β-mercaptoethanol, and 8 ng/mL bFGF. The medium was further supplemented with 8 ng/mL or 40 ng/mL of bFGF (Gibco); 40 ng/mL of bFGF and 15 ng/mL of SCF (R & D System); or 40 ng/mL of bFGF and 75 ng/mL of Flt3 ligand (R & D System). QBSF™-60 medium was supplemented with 0.1 mM β-mercaptoethanol, 1% non-essential amino acids (Gibco) and 40 ng/mL of bFGF. hES cells cultured in mEF conditioned medium were used as control in these experiments.

The hES cells were first passaged onto Matrigel coated plates using collagenase IV, and cultured for 2 days with conditioned medium. On day 2, the conditioned medium was replaced with 80% unconditioned ES medium plus 20% expansion medium. Cells were fed fresh daily and passaged weekly. The proportion of expansion medium was increased by 20% approximately every 2 days until the cells were completely weaned, and then grown until they had been passaged 6 more times.

**Figure 6** shows colonies of hES cell at the end of 6 passages (sufficient for full adaptation) in the following media: (A) mEF conditioned medium + bFGF (8ng/mL); (B) X-VIVO 10 + bFGF (40ng/mL); (C) X-VIVO 10 + bFGF (40ng/mL) + stem cell factor (SCF, Steel factor) (15 ng/mL); (D) X-VIVO 10+ bFGF (40ng/mL) + Flt3 ligand (75 ng/mL); (E) QBSF™-60 + bFGF (40ng/mL).

The following table shows the average total cell expansion per passage, for undifferentiated hES cells cultured for 4 passages in mEF conditioned medium, or for 7 passages in X-VIVO 10 or QBSF™-60.

| Table 3: Growth Rates for ES Cell Cultures | |
|---|---|
| Medium | Average Cell Expansion per Passage |
| mEF conditioned medium | 2.2 fold |
| X-VIVO 10 + bFGF (40ng/mL) | 6.0 fold |
| X-VIVO 10 + bFGF (40ng/mL) + SCF (15 ng/mL) | 8.2 fold |
| X-VIVO 10 + bFGF (40ng/mL) + Flt3 ligand (75 ng/mL) | 5.0 fold |
| QBSF-60 + bFGF (40ng/mL) | 6.4 fold |

The average expansion of cells per passage in X-VIVO 10 and OBSF™-60 was greater than the cells cultured in mEF conditioned medium culture. The cells in mEF conditioned medium were passaged on average every 7 days, while the cells in X-VIVO 10 and QBSF™-60 were passaged on average every 5 days. Thus, the rate of expansion in unconditioned X-VIVO 10 or QBSF™-60 was -3.2 to 5.2 times faster than in mEF conditioned ES medium.

**Figure 7** shows the gene expression profile of hTERT and Oct3/4. The RNA was isolated from the cells using High Pure RNA Isolation Kit (Roche Diagnostics) and evaluated by Taqman assay (real time RT-PCR). The gene expression in each of the test condition is plotted relative to expression in the control culture. Taking into consideration the instrument error and assay variability, differences in expression between the test and control samples are only significant if greater than 2-fold. The analysis shows expression of hTERT and Oct-3/4 decreases somewhat upon adaptation to unconditioned X-VIVO 10 or QBSF™-60 medium (first four bars in each set), but returns to standard levels when the cells are passaged back into mEF conditioned medium (last three bars in each set).

To confirm that cells cultured in unconditioned medium retain their pluripotency, embryoid bodies were formed and analyzed by immunocytochemistry for phenotypic markers representing each of the three germ layers. After passage 7 in expansion medium, the cells were dissociated into small clumps using 200 U/mL collagenase IV at 37°C for 10 min, placed in suspension culture in differentiation medium (DMEM + 10% FBS) for 4 days, then transferred onto poly-L-ornithine hydrobromide coated plates for a further 10 days. They were fixed in 4% paraformaldehyde, permeabilized, and labeled by immunocytochemistry.

**Figure 8** shows the results. hES cells passaged 7 times in unconditioned X-VIVO 10 medium stained for α-fetoprotein (representing endoderm); muscle actin (representing mesoderm), and β-tubulin III (representing ectoderm).

These results show that hES cells can be expanded in fresh (non-conditioned) media in a feeder-free environment at a rapid rate suitable for commercial production. The cells retain the morphology of undifferentiated hES cells, and can be differentiated into derivative cells representing all three germ layers.

### Example 6: Culture of hES cells in a defined system free of animal-based products

hES cells cultured in MEF-CM on Matrigel were passaged to a fresh (non-conditioned) serum free medium: X-VIVO 10 supplemented with Glutamine, non-essential amino acids and β-mercaptoethanol, plus 80 ng/mL human basic FGF on Matrigel, and then adapted to surfaces coated with human laminin. Alternatively, cryopreserved cells were directly thawed into the same medium containing 80 ng/mL hbFGF. The cells were passaged every 5-6 days using Collagenase IV.

**Figure 9** shows that cultures grown under these conditions were similar or better than cultures on Matrigel. (A) morphology for cells grown in mEF conditioned medium; (B) morphology fin defined medium on laminin; (C) Surface marker SSEA-4 expression in mEF-CM (H1 p62) or defined medium (H1 p34+28); (D) Expression of surface marker Tra-1-60 in mEF-CM or defined medium. Culture performance in the defined medium on laminin was superior: very large ES cell colonies were observed, with colonies representing about 80% of the culture. Levels of marker expression were as follows:

| Table 4: Marker Expression in Defined Culture Conditions | | | | | | |
|---|---|---|---|---|---|---|
| Passage no. | Culture medium | Surface marker expression | | Relaative gene expression | | |
| | | SSEA-4 | Tra-1-60 | hTERT | Oct3/4 | Cripto |
| *Experiment 1:* | | | | | | |
| H1p41 | Conditioned | 79% | 93% | 1.00 | 1.00 | 1.00 |
| H1p31+10 | Defined | 92% | 87% | 2.85 ± 0.58* | 0.74 ± 0.04 | 1.82 ± 0.62 |

| *Experiment 2:* | | | | | | |
|---|---|---|---|---|---|---|
| H1p44 | Conditioned | 81% | 91% | 1.00 | 1.00 | 1.00 |
| H1p34+11 | Defined | 77% | 84% | 1.11 ±0.38 | 0.57 ± 0.24 | 0.76 ± 0.39 |

| *Experiment 3:* | | | | | | |
|---|---|---|---|---|---|---|
| H1p47 | Conditioned | 78% | 92% | 1.00 | 1.00 | 1.00 |
| H1p35+12 | Defined | 80% | 86% | 2.00 ±0.15 | 0.86 ± 0.20 | 3.12 ± 0.91 |

| | | | | | | |
|---|---|---|---|---|---|---|
| * mean ± SD for 3 RT-PCR determinations | | | | | | |

Expression of other markers characteristic of undifferentiated hES cells was also comparable: Measured by real-time PCR, the levels of hTERT and Cripto were the same or greater in defined medium compared with mEF-CM, while the expression of Oct 3/4 was lower by about 28% (average of three experiments).

| Table 5. Cell expansion of H1 undifferentiated cells grown in defined system | | |
|---|---|---|
| Cultures | Standard | Defined |
| Experiment 1 | 2.16±0.86 (n=18) | 6.94±1.65(n=14) |
| Experiment 2 | 3.16±1.33 (n=16) | 6.75±2.27 (n=17) |
| Experiment 3 | 3.57±0.89 (n=18) | 5.29±1.30 (n=18) |

| | | |
|---|---|---|
| Standard system indicates MEF-CM-Matrigel with FGF at 8 ng/ml; Defined system indicates X-VIVO 10-Laminin with hbFGF at 80 ng/ml. The expansion fold is calculated as input cell number divided by output cell number. The seeding density was 5-8x10⁴ cells/cm². The cells were split every 5-7 days. | | |

**Figure 10** shows expression of gene products for Oct 3/4 and hTERT. Panels A and C: phase contrast images of hES cells (H1p34+14; H1p34+11) grown in defined medium on human laminin; Panels B and D: Oct 3/4 expression of the same fields. Panel E: TRAP analysis of telomerase activity for H1 hES cells grown in defined medium on laminin.

**Figure 11** shows teratomas generated by H1 hES cells grown in completely defined culture system at p34+11 (75 days). Panel A: Pigmented epithelium (endoderm); Panel B: Renal tissue (endoderm); Panel C: Mesenchymal tissue (mesoderm); Panel D: Neural tubes (ectoderm). All the images were taken at 200X. Tissues from three three-germ layers were observed, confirming that the cells retain their pluripotency.

One of the virtues of the culture medium described in this example is that hES cells previously grown in other conditions typically do not need to be weaned or adapted into the new system. For example, hES cells cultured in mEF-conditioned medium and cryopreserved can be directly cultured in a medium supplemented with Glutamine, non-essential amino acids, plus 80 ng/mL human basic FGF.

### Example 7 : Supplementation of Culture Media with TGFβ1

Undifferentiated hES cells were evaluated for their potential for growth in X-VIVO 10 to which hbFGF and TGFβ1 had been added.

Either H1 cells at passage 46 ("H1 p46") or H1 cells at passage 43 ("H1 p43") were seeded onto 6-well plates that had been coated with BD Matrigel™ Matrix Growth Factor Reduced (hereinafter "Matrigel-GFR") (BD, Bedford, MA) at a density between 5-8 x 10⁴ cells/cm². Each well contained about 4 ml of medium (X-VIVO 10 + 80 ng/ml hbFGF + 0.5ng/ml TGFβ1). The medium was exchanged for fresh medium every day after the first day. The cells were split every 5-7 days.

Culture 1 - the H1 p46 cells were passaged six times according to the above protocol (creating H1 p46+6 cells). There was an average 8.56 ± 1.41-fold expansion in cell number over the six passages (see Table 6). After the six passages, the expression levels of the surface markers Tra-1-60, SSEA-4, Oct-4, and Podocalyxin, as determined by flow cytometry, were within the normal range for undifferentiated H1 cells (see Table 7). The H1p46+6 cells were further passaged as above. At 8 passages (H1p46+8), the cells exhibited normal karyotype (see Table 8 and Figure 13), and at 9 passages (H1 p46+9), the cells exhibited normal morphology (see Figure 12 (A) and (B)).

Culture 2 - the H1 p43 cells were passaged seven times according to the above protocol (creating H1p43+7 cells). There was an average 9.73 ± 1.01-fold expansion in cell number over the seven passages (see Table 6). After the seven passages, the expression levels of the surface markers Tra-1-60, SSEA-4, Oct-4, and Podocalyxin, as determined by flow cytometry, were within the normal range for undifferentiated H1 cells (see Table 7). The karyotype of the H1p43 cells at passage 5 (H1p43+5) was normal (see Table 8).

| Table 6 | |
|---|---|
| Expansion of H1 hES cells grown in X-VIVO 10/ hbFGF/TGFβ/ Matrigel | |
| Cultures | Fold expansion* |
| Culture 1 | 8.56 ± 1.41 |
| Culture 2 | 9.73 ± 1.01 |
| * The fold expansion was calculated by dividing the output cell number by the input cell number for each passage and then averaging the data for all passages. | |

| Table 7 | | | | |
|---|---|---|---|---|
| Surface marker expression on H1 hES cells grown in X-VIVO 10/hbFGF/TGFβ1/Matrigel | | | | |
| | | | | |
| Cultures | Tra-1-60 | SSEA-4 | Oct-4 | Podocalyxin |
| Culture 1 | 88.9% | 88.9% | 62.8% | 68.2% |
| Culture 2 | 78.8% | 88.0% | 36.0% | 77.0% |

| Table 8 | | |
|---|---|---|
| Karyotype of H1 hES cells grown in X-VIVO 10/ hbFGF/ TGFβ/ Matrigel | | |
| Cultures | Passage # | Karyotype |
| Culture 1 | H1p46+8 | 46, XY |
| Culture 2 | H1p43+5 | 46, XY |

## Claims

1. A medium for culturing human embryonic stem (hES) cells in vitro such that they proliferate without differentiating, comprising:
- an isotonic buffer
- a protein nutrient, comprising serum, serum replacement, albumin, or essential or non-essential amino acids;
- lipids, fatty acids, or cholesterol, either as artificial additives or as the HDL or LDL extract of serum;
- added fibroblast growth factor at a concentration of at least 40ng/ml; and
- an added transforming growth factor (TGF) selected from the group consisting of TGFβ1, TGFβ2, and TGFβ3.

2. A medium as claimed in claim 1, wherein the medium further comprises added nucleosides or nucleotides.

3. A medium as claimed in any preceding claim, wherein the medium further comprises added insulin.

4. A medium as claimed in any preceding claim, wherein the medium further comprises added transferrin.

5. A medium as claimed in any preceding claim, wherein the fibroblast growth factor is recombinant basic fibroblast growth factor.

6. A medium as claimed in claim 5, wherein the basic fibroblast growth factor is recombinant basic human fibroblast growth factor.

7. A medium as claimed in any preceding claim, wherein the medium contains between 40 and 200 ng/ml basic FGF.

8. A medium as claimed in any preceding claim, wherein the medium contains at least 80 ng/ml basic FGF.

9. A medium as claimed in any preceding claim, wherein the medium further comprises added stem cell factor (SCF).

10. A medium as claimed in any preceding claim , wherein the medium further comprises added Flt3 ligand.

11. A medium as claimed in claim 1, wherein the medium contains at least 0.5ng/ml TGFβ1.

## Patentansprüche

1. Medium zum Züchten humaner embryonaler Stamm- (hES - *human embryonic stem*) Zellen in vitro, derart, dass sie proliferieren, ohne sich zu differenzieren, welches Folgendes umfasst:
- einen isotonischen Puffer;
- einen Proteinnährstoff, der Serum, Serumersatz, Albumin oder essentielle oder nichtessentielle Aminosäuren umfasst;
- Lipide, Fettsäuren oder Cholesterin, entweder als künstliche Zusatzstoffe oder als der HDL- oder LDL-Extrakt von Serum;
- zugegebenen Fibroblasten-Wachstumsfaktor (FGF - *fibroblast growth factor*) mit einer Konzentration von mindestens 40 ng/ml; und
- einen zugegebenen transformierenden Wachstumsfaktor (TGF - *transforming growth factor*) ausgewählt aus der Gruppe bestehend aus TGFβ1, TGFβ2 und TGFβ3.

2. Medium nach Anspruch 1, wobei das Medium ferner zugegebene Nukleoside oder Nukleotide umfasst.

3. Medium nach einem der vorhergehenden Ansprüche, wobei das Medium ferner zugegebenes Insulin umfasst.

4. Medium nach einem der vorhergehenden Ansprüche, wobei das Medium ferner zugegebenes Transferrin umfasst.

5. Medium nach einem der vorhergehenden Ansprüche, wobei der Fibroblasten-Wachstumsfaktor rekombinanter basischer Fibroblasten-Wachstumsfaktor ist.

6. Medium nach Anspruch 5, wobei der basische Fibroblasten-Wachstumsfaktor rekombinanter basischer humaner Fibroblasten-Wachstumsfaktor ist.

7. Medium nach einem der vorhergehenden Ansprüche, wobei das Medium zwischen 40 und 200 ng/ml basischen FGF enthält.

8. Medium nach einem der vorhergehenden Ansprüche, wobei das Medium mindestens 80 ng/ml basischen FGF enthält.

9. Medium nach einem der vorhergehenden Ansprüche, wobei das Medium ferner zugegebenen Stammzellfaktor (SCF - *stem cell factor*) umfasst.

10. Medium nach einem der vorhergehenden Ansprüche, wobei das Medium ferner zugegebenen Flt3-Liganden umfasst.

11. Medium nach Anspruch 1, wobei das Medium mindestens 0,5 ng/ml TGFβ1 enthält.

## Revendications

1. Milieu pour la culture in vitro de cellules souches embryonnaires humaines (hES), de sorte qu'elles prolifèrent sans se différencier, comprenant :
- un tampon isotonique
- un nutriment protéique, comprenant du sérum, un substitut de sérum, de l'albumine ou des acides aminés essentiels ou non essentiels ;
- des lipides, des acides gras, ou du cholestérol, soit sous forme d'additifs artificiels soit sous forme d'extrait de sérum de HDL ou LDL ;
- un facteur de croissance de fibroblastes ajouté à une concentration d'au moins 40 ng/mL ; et
- un facteur de croissance transformant (TGF) ajouté, sélectionné dans le groupe consistant en TGFβ1, TGFβ2 et TGFβ3.

2. Milieu selon la revendication 1, dans lequel le milieu comprend en outre des nucléosides ou des nucléotides ajoutés.

3. Milieu selon l'une quelconque des revendications précédentes, dans lequel le milieu comprend en outre de l'insuline ajoutée.

4. Milieu selon l'une quelconque des revendications précédentes, dans lequel le milieu comprend en outre de la transferrine ajoutée.

5. Milieu selon l'une quelconque des revendications précédentes, dans lequel le facteur de croissance des fibroblastes est un facteur de croissance des fibroblastes basique recombinant.

6. Milieu selon la revendication 5, dans lequel le facteur de croissance des fibroblastes basique est un facteur de croissance des fibroblastes humain basique recombinant.

7. Milieu selon l'une quelconque des revendications précédentes, dans lequel le milieu contient entre 40 et 200 ng/mL de FGF basique.

8. Milieu selon l'une quelconque des revendications précédentes, dans lequel le milieu contient au moins 80 ng/mL de FGF basique.

9. Milieu selon l'une quelconque des revendications précédentes, dans lequel le milieu comprend en outre un facteur de cellules souches (SCF) ajouté.

10. Milieu selon l'une quelconque des revendications précédentes, dans lequel le milieu comprend en outre le ligand Flt3 ajouté.

11. Milieu selon la revendication 1, dans lequel le milieu contient au moins 0,5 ng/mL de TGFP1.
